(19) Europäisches Patentamt / European Patent Office / Office européen des brevets

(11) **EP 3 052 198 B1**

(12) **FASCICULE DE BREVET EUROPEEN**

(45) Date de publication et mention de la délivrance du brevet:
**21.03.2018 Bulletin 2018/12**

(21) Numéro de dépôt: **14780461.1**

(22) Date de dépôt: **01.10.2014**

(51) Int Cl.:
*A61Q 19/00* (2006.01)  *A61K 8/92* (2006.01)
*A61K 8/97* (2017.01)  *A61Q 19/08* (2006.01)

(86) Numéro de dépôt international:
**PCT/EP2014/071006**

(87) Numéro de publication internationale:
**WO 2015/049265 (09.04.2015 Gazette 2015/14)**

(54) **COMPOSITION HUILEUSE CONTENANT UN EXTRAIT D'HEMEROCALLE ET UTILISATIONS**

ÖLIGE ZUBEREITUNG ENTHALTEND EINEN EXTRAKT VON HEMEROCALLIS-PFLANZEN UND SEINE VERWENDUNG

OILY COMPOSITION COMPRISING AN EXTRACT OF HEMEROCALLIS AND ITS USE

(84) Etats contractants désignés:
AL AT BE BG CH CY CZ DE DK EE ES FI FR GB GR HR HU IE IS IT LI LT LU LV MC MK MT NL NO PL PT RO RS SE SI SK SM TR

(30) Priorité: **02.10.2013 FR 1359546**

(43) Date de publication de la demande:
**10.08.2016 Bulletin 2016/32**

(73) Titulaire: **Société De Recherche Cosmétique S.à.r.L.**
**2314 Luxembourg (LU)**

(72) Inventeurs:
• **LECONTE, Nadine**
  **F-92600 Asnieres sur Seine (FR)**
• **ROSSIGNOL-CASTERA, Anne**
  **F-34160 Restinclieres (FR)**

(74) Mandataire: **Goulard, Sophie**
  **Ipsilon**
  **Le Centralis**
  **63, avenue du Général Leclerc**
  **92340 Bourg-la-Reine (FR)**

(56) Documents cités:
**FR-A1- 2 943 684**  **JP-A- 2006 143 670**
**JP-A- 2012 012 318**

• **DATABASE GNPD [Online] MINTEL; novembre 2009 (2009-11), KAO: "Cream est Eternal Flow", XP002722293, Database accession no. 1211158**
• **DATABASE GNPD [Online] MINTEL; juillet 2013 (2013-07), Mantong, South Korea: "Transformation Cleansing Milk Oil", XP002722302, Database accession no. 2122962**
• **DATABASE GNPD [Online] MINTEL; février 2012 (2012-02), Tony Moly: "BB Cream SPF 30 PA++", XP002722328, Database accession no. 1737909**
• **NAMSA N D ET AL: "An ethnobotanical study of traditional anti-inflammatory plants used by the Lohit community of Arunachal Pradesh, India", JOURNAL OF ETHNOPHARMACOLOGY, ELSEVIER IRELAND LTD, IE, vol. 125, no. 2, 7 septembre 2009 (2009-09-07), pages 234-245, XP026498478, ISSN: 0378-8741, DOI: 10.1016/J.JEP.2009.07.004 [extrait le 2009-07-14]**

**Description**

La présente invention se rapporte au domaine technique général des produits cosmétiques à usage topique, utilisables notamment sur la peau.

Plus précisément, la présente invention est relative à une nouvelle composition huileuse à base d'un extrait lipophile d'hémérocalle, aux compositions cosmétiques comprenant une telle composition huileuse, à un procédé cosmétique non thérapeutique pour protéger la peau des agressions de l'environnement, prévenir et combattre les signes du vieillissement cutané de la peau mettant en oeuvre de telles compositions cosmétiques, ainsi qu'à l'utilisation non thérapeutique d'une composition cosmétique à application topique comprenant un extrait lipophile d'hémérocalle pour prévenir les signes du vieillissement cutané.

La peau est un véritable organe comprenant plusieurs couches intégrées, allant de la couche superficielle, l'épiderme, jusqu'aux couches plus profondes, le derme et l'hypoderme, et chacune de ces couches possède des propriétés spécifiques permettant à l'ensemble de réagir et de s'adapter aux conditions de son environnement.

L'épiderme est principalement composé de kératinocytes (90% des cellules épidermiques) qui synthétisent la kératine, de mélanocytes (2 à 3% des cellules épidermiques) responsables de la pigmentation de la peau, et des cellules de Langerhans qui jouent un rôle immunologique. L'épiderme, dont l'épaisseur est variable selon les différentes parties du corps, constitue la couche externe de la peau et par conséquent il joue un rôle fondamental pour assurer la protection et le maintien d'une bonne trophicité. C'est pourquoi de nombreuses compositions ont été mises au point afin de le protéger, notamment des agressions de l'environnement.

Le derme est la couche la plus épaisse, riche en nerfs, en vaisseaux sanguins et en glandes sudoripares, et se compose principalement de collagène, d'élastine et de protéoglycanes. Ces trois types de molécules sont synthétisés par les fibroblastes dermiques. Les fibres de collagène, qui représentent 70% du poids sec du derme, assurent la résistance mécanique et la texture de la peau, l'élastine est responsable de l'élasticité, et les protéoglycanes jouent un rôle majeur de structure et d'hydratation de la peau. D'autres cellules comme les macrophages et les leucocytes sont également présentes dans la couche du derme.

L'hypoderme, qui est la couche la plus profonde de la peau, contient les adipocytes qui produisent des lipides pour que le tissu sous-cutané fabrique une couche grasse protégeant les muscles, les os et les organes internes contre les chocs.

Le vieillissement de la peau peut être intrinsèque, ou extrinsèque c'est-à-dire provoqué par l'environnement, y compris les agressions climatiques, qui peuvent notamment contribuer à accélérer la dégradation du collagène du derme, et en particulier l'exposition au soleil, les variations de températures et les radicaux libres qui sont considérés comme une des causes du vieillissement tissulaire, en particulier en ce qui concerne l'apparition des rides de la peau. L'introduction d'antioxydants dans de nombreuses compositions cosmétiques à application topique telles que crèmes, laits, sérums, etc..., a pour objectif de combattre les effets néfastes de ces radicaux libres.

Un radical libre est un atome ou une molécule qui a gagné ou perdu un électron. Un exemple d'intérêt biologique est la molécule de dioxygène qui gagne un électron lors de la respiration cellulaire pour conduire au radical superoxyde. C'est ce nombre impair d'électrons qui rend la molécule instable. Celle-ci n'aura alors de cesse de capter ou céder un électron à une autre molécule de son entourage, propageant ainsi le phénomène. Lorsqu'elle se produit dans l'organisme, cette réaction en chaîne est communément appelée stress oxydant. Elle provoque de nombreux dégâts dans les tissus, les organes, et peut modifier certains gènes. Elle est impliquée dans de nombreuses maladies comme la cataracte, l'arthrite, les maladies cardio-vasculaires ou les cancers.

L'organisme aérobie possède heureusement un système de défense efficace contre le stress oxydant. En effet, de nombreux antioxydants naturels (enzymes et vitamines) aux propriétés anti-radicalaires sont soit produits par l'organisme, soit tirés de l'alimentation. Ainsi, la vitamine C inhibe les radicaux libres à l'intérieur de la cellule alors que la vitamine E et les caroténoïdes jouent le même rôle au niveau de la membrane cellulaire.

Le vieillissement cellulaire peut se définir comme la perte progressive de la capacité de l'organisme à maintenir l'équilibre entre radicaux libres et antioxydants. Au niveau de la peau, les radicaux libres induisent les marques du temps qui s'écoule : le flétrissement de la peau, le relâchement cutané, les rides, les taches pigmentaires... et également les cancers de la peau.

Il est donc possible de palier ce déséquilibre par l'application sur la peau de compositions cosmétiques ayant des propriétés anti-radicalaires afin de lutter contre l'apparition des signes du vieillissement.

Les produits d'origine naturelle ou contenant des composés naturels sont par ailleurs de plus en plus appréciés par les consommateurs et consommatrices de produits cosmétiques. C'est pourquoi on trouve sur le marché des produits cosmétiques de plus en plus de produits contenant des substances naturelles ou d'origine naturelle et présentant des propriétés anti-radicalaires.

De telles compositions contiennent classiquement au moins une substance anti-oxydante, en particulier des molécules complexes d'origine végétale telles que par exemple les polyphénols, les flavonoïdes, les anthocyanosides, les caroténoïdes, etc...

Ces molécules ne sont toutefois pas toujours assez stables dans le temps et perdent ainsi progressivement leur efficacité.

Il est donc difficile, à l'heure actuelle, d'obtenir des compositions cosmétiques contenant un extrait végétal d'origine naturelle et dont les propriétés restent stables dans le temps, alors même que le besoin semble important.

Hémérocalle est le nom commun donné à la fleur du genre *Hemerocallis* qui appartient à la famille des liliacées.

Elle est aussi appelée Lys d'un jour. En effet, elle doit son nom au grec ἡμέρα (Hemera) «jour» et

$$\kappa\alpha\lambda\acute{o}\varsigma$$

(kalos) «beauté». On l'appelle lys d'un jour, car sa fleur est éphémère et ressemble au lys, mais le feuillage est totalement différent. La fleur de l'hémérocalle ne vit qu'une seule journée, mais de nouvelles fleurs se succèdent pendant plusieurs semaines. Les fleurs se fanent à la tombée du jour et laissent la place à d'autres dès le lendemain matin.

Les Hémérocalles sont connues et cultivées depuis des siècles. Originaires d'Asie, les premières espèces étaient déjà cultivées, décrites et peintes à l'époque de Confucius (551 - 479 Av JC).

*Hemerocallis fulva* est connue, notamment en médecine traditionnelle chinoise, pour ses effets calmants, cicatrisants et bactéricides. Elle est également utilisée dans le traitement de la dépression, de l'inflammation, de l'insomnie et des furoncles.

En particulier, la demande de brevet US2011/0076349 décrit une composition à administrer par voie orale contenant, à titre de principe actif, un extrait aqueux des parties aériennes *d'Hemerocallis fulva* et ayant des effets antidépresseurs ou défatiguants basés sur l'amélioration du sommeil.

Les études effectuées par la Demanderesse ont montré qu'un extrait huileux d'Hémérocalle, plus précisément d'*Hemerocallis fulva,* présente des propriétés anti-radicalaires utiles pour la protection de la peau et dans la prévention des signes du vieillissement cutané.

La présente invention a donc pour premier objet une composition huileuse à base d'un extrait d'hémérocalle, ladite composition étant caractérisée en ce qu'elle comprend un extrait lipophile *d'Hemerocallis fulva,* et un véhicule huileux comprenant au moins une huile végétale (huile vectrice).

Les compositions cosmétiques à application topique comprenant une telle composition huileuse à titre d'agent anti-oxydant constituent un autre objet de l'invention. Elles présentent d'excellentes propriétés anti-radicalaires utilisables en cosmétique pour les soins de la peau, plus particulièrement pour protéger la peau des agressions de l'environnement et pour prévenir les signes du vieillissement cutané. Les propriétés anti-radicalaires des compositions cosmétiques incorporant la composition huileuse conforme à l'invention sont de plus particulièrement stables dans le temps.

Les essais effectués par la Demanderesse ont montré que la composition huileuse conforme à l'invention permet de réduire le niveau d'oxydation intracellulaire induit par les UV sur des épidermes reconstruits et que cette activité se manifeste de façon dose-dépendante dans la gamme de concentrations testées. De plus, ces essais ont également montré que lorsqu'une composition cosmétique comprenant une composition huileuse conforme à l'invention est appliquée avant une irradiation UV, celle-ci protège les cellules cutanées des effets délétères induits par les UV de façon plus efficace que la vitamine E pure. En effet, la composition huileuse conforme à l'invention est composée d'une combinaison d'antioxydants qui agissent de façon synergique contre les phénomènes oxydatifs auxquels les cellules cutanées sont exposées. Elle apporte à la peau à la fois des antioxydants apolaires tels que la vitamine E et des caroténoïdes (lutéine, zéaxanthine), mais aussi des antioxydants plus polaires tels que les polyphénols (catéchines, quercétine, rutine, acides chlorogéniques) ou la vitamine C. Cette combinaison d'agents antioxydants, véhiculée par l'huile vectrice, permet une meilleure protection contre l'oxydation responsable du vieillissement cutané.

De plus, aux doses utilisées, les essais effectués ont montré que la composition huileuse utilisée dans la présente invention ne présente aucune cytotoxicité.

L'invention a donc également pour autre objet, un procédé cosmétique non thérapeutique pour protéger la peau des agressions de l'environnement, prévenir et combattre les signes du vieillissement cutané, consistant à appliquer sur les zones de la peau concernées au moins une composition cosmétique telle que définie ci-dessus, c'est-à-dire une composition cosmétique topique contenant une quantité efficace de la composition huileuse conforme à l'invention.

Enfin, l'invention a pour objet l'utilisation non thérapeutique d'une composition cosmétique à application topique comprenant un extrait lipophile d'hémérocalle pour protéger la peau des agressions de l'environnement, prévenir et combattre les signes du vieillissement cutané.

Les études effectuées par la demanderesse ont montré que l'on peut utiliser toutes les parties aériennes de la plante, aussi bien les tiges, les feuilles et les fleurs. L'espèce *Hemerocallis fulva* est facilement disponible et la conservation des plantes ne soulève généralement pas de difficulté technique.

La composition huileuse conforme à l'invention peut être préparée selon le procédé d'extraction décrit dans la demande internationale WO 2010/112760 consistant, dans une première étape, à mélanger et imprégner la plante, préalablement séchée et broyée, dans au moins une huile végétale naturelle à une température supérieure à son point de fusion, puis à chauffer le mélange à haute température pendant une durée très courte, et à former une microdispersion à une température supérieure à la température de fusion de l'huile végétale. Toutes ces étapes se font de préférence à l'abri de l'air, plus particulièrement à l'abri de l'oxygène pour éviter toute réaction d'oxydation de l'extrait. On peut par exemple travailler sous atmosphère d'azote, dans un réacteur clos avec extraction continue de l'oxygène par un flux d'azote, ou encore sous vide.

Les huiles végétales naturelles utilisables dans ce procédé sont de préférence choisies parmi l'huile d'avocat, l'huile de rosier muscat, l'huile de noisette, l'huile de colza, l'huile de macadamia, l'huile d'argan, l'huile de tournesol, l'huile de cameline, l'huile de bourrache, huile d'amande douce, huile de carthame, huile de calophyllum et leurs mélanges.

Selon, une forme de réalisation préférée de l'invention, l'huile végétale est choisie parmi l'huile d'avocat, l'huile de rosier muscat et leurs mélanges.

Selon une forme de réalisation tout particulièrement préférée de l'invention, le véhicule huileux comprend un mélange d'huile d'avocat et d'huile de rosier muscat.

Les huiles végétales utilisées selon l'invention, et en particulier les huiles d'avocat et de rosier muscat, sont des produits disponibles dans le commerce.

L'avocat est le fruit de l'avocatier (*Persea americana*), un arbre originaire d'Amérique du Nord (Mexique). Riche en acide oléique, en caroténoïdes et en éléments nutritifs, l'huile d'avocat est extraite de la pulpe du fruit (chair) car paradoxalement, le noyau ne contient que très peu de corps gras.

L'huile de rosier muscat est extraite des graines du cynorhodon (un « faux » fruit) du rosier muscat qui est un églantier sauvage. Elle est l'une des huiles végétales les plus riches en acides gras poly-insaturés essentiels (acides linoléique et linolénique) et contient des insaponifiables. Elle a des propriétés régénérantes.

L'utilisation de ces huiles végétales comme solvant de l'extrait de d'hémérocalle présente l'avantage d'être compatible avec les normes des produits certifiés Ecocert s'appliquant aux produits cosmétiques à la fois écologiques et biologiques.

La première étape du procédé ci-dessus, consistant à imprégner la plante, de préférence broyée, se déroule à température ambiante, ou à chaud, à une température supérieure à celle de fusion des huiles utilisées. On travaille de préférence à température ambiante, de l'ordre de 25°C, les huiles végétales, et en particulier les huiles d'avocat et de rosier muscat, étant liquides à cette température.

Selon une forme de réalisation préférée, le broyage de la plante est effectué à une température d'environ -80°C, jusqu'à obtention d'une poudre présentant une granulométrie moyenne inférieure à 100 $\mu$m environ.

La deuxième étape comporte un chauffage à une température élevée, par exemple de l'ordre de 140 à 170°C, pendant une courte durée, généralement inférieure à 5 minutes. Le temps de montée en température doit aussi être très court. La technique du chauffage par micro-ondes est bien adaptée à ces conditions. La technique des micro-ondes facilite aussi la troisième étape de microdispersion des particules dans la ou les huiles végétales utilisées, avec rupture des membranes cellulaires. Cette troisième étape peut aussi comprendre un traitement par cavitation ultrasonore, de préférence dans un réacteur fermé équipé d'un générateur d'ultrasons à basse fréquence. Cette troisième étape peut éventuellement être réalisée simultanément avec les deux autres, l'ensemble des étapes, ou chacune d'elles, pouvant être réalisé plusieurs fois si nécessaire.

A la fin de l'extraction, l'extrait lipophile est de préférence filtré sur un filtre ayant une porosité inférieure à environ 5 $\mu$m, de façon à obtenir un extrait lipophile limpide.

Ce procédé est particulièrement efficace sur une matière première telle que l'hémérocalle contenant entre 85 et 90 % d'eau avant broyage et séchage. Après séchage, avant l'étape d'imprégnation dans la ou les huiles végétales, la teneur en eau est ramenée en dessous de 12 %.

Lorsque l'extraction est réalisée en utilisant un mélange d'huile d'avocat et de rosier muscat, on obtient un extrait lipophile de couleur vert foncé ayant une densité relative à 20°C variant de 0,900 à 0,990 g/cm$^3$ environ (NF ISO 6883), soluble dans les huiles et phases grasses, et ayant les caractéristiques suivantes :

- Teneur en eau et en volatils (NF EN ISO 662) : max. 0,20 % $\pm$ 0,04 % ;

- Acidité oléique (NF EN ISO 660) : max. 3,0% $\pm$ 0,6% ;

- Indice de peroxyde (NF EN ISO 3960) : max 10,0 $\pm$ 4,0 méqO$_2$/kg ;

- Teneur en phénols (dosages par HPLC) :

  * Catéchines : 150 mg/kg

* Quercétine : 250 mg/kg

* Rutine : 150 mg/kg

* Acide chlorogénique : 416 mg/kg.

- Teneur totale en caroténoïdes (lutéine et zéaxanthine) : 5,1 mg/kg de composition huileuse (mesuré par HPLC) ;

- Teneur en β-carotène : 8,6 mg/kg de composition huileuse (mesuré par HPLC) ;

- Composition en acides gras de la composition huileuse :

* acide palmitique 6-26 %

* Acide palmitoléïque 1,5-10 %

* Acide stéarique max. 2 %

* Acide oléique 30-65 %

* Acide linoléique 13-27 %

* Acide α-linolénique 5-14 %

* Acide arachidique max. 1 %

* Acide érucique max. 1 %

De plus, la technique utilisée permet d'obtenir une composition huileuse bien plus concentrée en principes actifs qu'un simple macérât huileux. Ainsi, suivant l'invention on associe les avantages d'une huile végétale et de molécules hydrophiles (flavonoïdes, composés phénoliques). Enfin, le véhicule huileux évite de devoir ajouter un conservateur à la composition, celui-ci permettant en effet de stabiliser dans le temps les propriétés anti-radicalaires de l'extrait lipophile d'hémérocalle et donc l'efficacité des compositions cosmétiques la renfermant.

Selon une forme de réalisation préférée de l'invention, le véhicule huileux comprend un mélange d'huile d'avocat et d'huile de rosier muscat. Dans ce cas, le rapport massique huile d'avocat/huile de rosier muscat au sein de la composition huileuse varie de préférence de 4/1 à 1/1 environ, et encore plus préférentiellement de 2/1 à 1/1.

Au sein de la composition huileuse conforme à l'invention, la teneur en extrait lipophile d'hémérocalle (molécules lipophiles extraites de la plante hémérocalle, soit principalement des caroténoïdes et du β-carotène) peut varier de 5 à 20 ppm, et plus préférentiellement, de 15 à 20 ppm.

La composition cosmétique à application topique selon l'invention est caractérisée en ce qu'elle comprend, à titre d'agent anti-oxydant, au moins une composition huileuse conforme à l'invention et telle que définie précédemment.

La teneur en composition huileuse dans la composition cosmétique peut varier généralement de 0,01 à 50 % en masse, de préférence de 0,1 à 10% environ en masse, et encore plus préférentiellement de 0,5 à 4,0 % environ en masse, par rapport à la masse totale de la composition.

Le choix de la quantité de composition huileuse dans la composition peut être fait en fonction de l'utilisation envisagée. Pour un traitement prolongé, on utilise de préférence des doses plus faibles, sous forme de lait ou de crème dosée à environ 0,1 à 2 %, tandis qu'un traitement ponctuel peut nécessiter des doses plus élevées, par exemple un gel ou un sérum dosé entre 3 et 10 %.

Ainsi, ces compositions topiques peuvent être utilisées avantageusement en cosmétologie pour la prévention des signes du vieillissement cutané.

Les compositions selon l'invention peuvent contenir, outre la composition huileuse conforme à l'invention, un ou plusieurs actifs secondaires renforçant ou complétant avantageusement son activité, et compatibles, c'est-à-dire non susceptibles de réagir les uns avec les autres ou de masquer ou limiter ses effets.

Plus particulièrement, les actifs secondaires peuvent être choisis parmi un agent anti-âge complémentaire, ou parmi l'acétyl tetrapeptide-5 (CAS N° 820959-17-9), un extrait de plancton, un extrait de maca qui a un effet sur la prévention de dérèglements liés à une déficience de la microcirculation cutanée et au stress oxydatif qui peut en résulter, un extrait de pétales de coquelicot agissant sur la nutrition cellulaire, une combinaison d'extraits d'anchuse, de coquelicot et de passiflore qui, en association, ont un effet dans la prévention des signes du vieillissement cutané, un extrait de graines de mimosa, un extrait de pétales de souci, les cellules de cacao, la vitamine C, la vitamine E et l'hexylrésorcinol.

L'agent anti-âge complémentaire peut être par exemple un lipide tel que le géranyl géranyl isopropanol (Juvinity®) qui réduit la formation des peroxydes intracellulaires et retarde ainsi le vieillissement cellulaire.

Les compositions cosmétiques de la présente invention possèdent une excellente tolérance cutanée, notamment en raison du film lipidique résultant du support huileux (huiles végétales, en particulier huile d'avocat et huile de rosier muscat), elles ne présentent aucune phototoxicité et leur application sur la peau, pour des périodes de temps prolongées, n'implique aucun effet systémique.

Les compositions conformes à la présente invention peuvent se présenter sous les formes galéniques classiquement utilisées pour une application topique, c'est-à-dire sous forme de gel, d'émulsion (en particulier crème ou lait), d'émulsion biphasique huile-dans-eau ou eau-dans-huile, d'huile corporelle, de masque, de pommade, d'onguent, de lotion, de solution concentrée, de nanocapsules, de liposomes ou de bâtons protecteurs pour les lèvres, lesdites formes galéniques contenant en outre des excipients et supports usuels et acceptables sur le plan cosmétologique. Elles sont utilisées de préférence sous forme de crèmes, de sérums, de lotion ou de gel.

Ces formes d'administration par voie topique sont préparées par les techniques usuelles, et par exemple, dans le cas d'une crème, par dispersion d'une phase grasse dans une phase aqueuse pour obtenir une émulsion huile-danseau, ou inversement pour préparer une émulsion eau-dans-huile. Dans le cas de crèmes, on peut utiliser des émulsions à structure lamellaire obtenues avec des lécithines hydrogénées ou des sucro-esters, contenant peu de produits éthoxylés ou n'en contenant pas du tout.

Les compositions cosmétiques suivant l'invention peuvent aussi prendre la forme de lotion ou solution dans laquelle les extraits sont sous forme encapsulée dans des microsphères. Les microsphères suivant l'invention peuvent par exemple être constituées de corps gras, d'agar et d'eau. La composition huileuse conforme à l'invention peut être incorporée dans des vecteurs de type liposomes, glycosphères, dans des chylomicrons, des macro-, micro-, nanoparticules ainsi que les macro-, micro- et nanocapsules et aussi être adsorbés sur des polymères organiques poudreux, les talcs, bentonites et autres supports minéraux.

Ces émulsions jouissent d'une bonne stabilité et peuvent être conservées pendant le temps nécessaire pour l'utilisation à des températures comprises entre 0 et 50 °C sans qu'il y ait sédimentation des constituants ou séparation des phases.

Les compositions topiques selon l'invention peuvent comprendre des excipients appropriés pour une application topique externe, en particulier des excipients acceptables sur le plan cosmétologique. Ces excipients appropriés pour la formulation sont bien connus de l'homme du métier et comprennent en particulier des agents de pénétration tels que le phytantriol, l'octyl dodécanol et l'escine ; les épaississants tels que les gommes naturelles et les polymères de synthèse ; les tensioactifs ; les émollients tels que l'octanoate de cétéaryle, le myristate d'isopropyle, l'isononanoate de cétéaryle, la diméthicone, la cyclométhicone, le 3-diisostéarate de polyglycéryle, le polyisobutène hydrogéné, l'alcool cétylique, le palmitate cétylique, le phosphate cétylique ; les émulsionnants tels que des dérivés de polyglycérol ; les conservateurs tels que le phénoxyéthanol et l'acide déhydroacétique ; les colorants ; les parfums ; etc...

Comme autres ingrédients utilisables dans les compositions de l'invention, on peut citer les agents hydratants tels que la glycérine, le butylène glycol, le sel de sodium de l'acide pyrrolidone carboxylique (sodium PCA), ainsi que des associations de dérivés glucosiques à effet hydratant et restructurant comme le produit Aquaxyl® (xylitylglucoside et anhydroxylytol et xylitol), et également les vitamines antioxydantes telles que la vitamine E, par exemple l'acétate de tocophérol ou le tocotriénol, la vitamine C, les polyphénols naturels.

On peut aussi ajouter à la composition des glucides choisis principalement parmi le glucose, le glycogène et le tréhalose, ou encore un palmitoyl pentapeptide-3 tel que le Matrixyl® ou des dérivés tels que le palmitoyl GHK (possédant la chaîne Glycyl-Histidyl-Lysine) et le palmitoyl GQPR (Glycyl-Glutamyl-Prolyl-Arginine) ou le palmitoyl VGVAPG (Valyl-Glycyl-Valyl-Alanyl-Prolyl-Glycine) associé à un céramide-2, ainsi que d'une manière générale toute association avec un céramide.

On peut également ajouter à la composition des agents de protection contre les rayons ultraviolets, et par exemple des filtres solaires UV-A et UV-B hydrophiles ou lipophiles, ou des pigments formant écran anti-ultraviolet.

Les filtres solaires peuvent être choisis par exemple parmi la benzophénone ou un dérivé de benzophénone tel que la 2-hydroxy-4-méthoxy-benzophénone (Eusolex® 4360), ou un ester d'acide cinnamique et plus particulièrement le méthoxycinnamate d'octyle (Eusolex® 2292), le méthoxycinnamate d'éthyl-2-hexyle (Parsol MCX®), ou encore un cyano-$\beta,\beta$-diphénylacrylate tel que l'octocrylène (Eusolex® OCR), le 4-méthylbenzylidène camphre (Eusolex 6300®), et des dérivés du dibenzoylméthane tels que le 4-isopropyl dibenzoylméthane (Eusolex 8020), le t-butyl-méthoxy dibenzoylméthane (Parsol 1789®), et le 4-méthoxy-dibenzoylméthane.

Les pigments peuvent être choisis par exemple parmi le dioxyde de titane, l'oxyde de zinc et l'oxyde de zirconium.

L'utilisation cosmétique de la composition cosmétique conforme à l'invention comprend tous les soins du corps et de la peau y compris les produits solaires, protecteurs et bronzants, les produits anti-âge, anti-séborrhéiques, toniques, les produits assurant l'amélioration de l'aspect de la peau y compris le traitement acnéique et les rougeurs cutanées.

Les exemples suivants illustrent l'invention plus en détail sans en limiter la portée. Dans tous les exemples de compositions qui suivent, les parties sont exprimées en masse, sauf indication contraire.

## EXEMPLES

### EXEMPLE 1 : Préparation d'une composition huileuse comprenant un extrait lipophile d'hémérocalle

Une composition huileuse à base d'extrait lipophile d'*Hemerocallis fulva* a été préparée comme indiqué ci-après.

On a utilisé l'ensemble des parties aériennes de la plante entière, préalablement séchée, puis broyée à froid. La technique du séchage/broyage à froid est préférable car elle permet de préserver les anti-oxydants présents dans la plante. Suivant cette technique, les plantes sont soumises à un premier broyage, puis à une déshydratation à température ambiante à l'abri de la lumière, de manière à ramener la teneur en eau vers 10%, et broyées à froid, à une température de -80°C, au moyen d'un broyeur à couteaux.

La teneur en eau était inférieure à 12 % après broyage et séchage à froid, ce pourcentage pouvant notamment varier de 8,5 à 12 % environ.

20 kg d'hémérocalle ainsi séchée et broyée, sous forme de poudre fine et homogène, ont été mélangés à 60 kg d'huile d'avocat et 20 kg d'huile de rosier muscat (rapport massique huile d'avocat/huile de rosier muscat = 3/1). Le rapport massique plante / huile était de 0,20.

Le mélange a ensuite été chauffé rapidement par micro-ondes à une puissance de 10000 à 30000 Watts/ kg de mélange, pendant une durée inférieure à 10 minutes, sous flux d'azote, la température maximale atteinte étant comprise entre 80 et 200°C.

Le mélange a ensuite été traité par ultrasons (100 kHz environ), sous azote et sous agitation, pendant 10 à 20 minutes environ. Le mélange d'huiles vectrices contenant l'extrait lipophile d'*Hemerocallis fulva* a été séparé par centrifugation.

### EXEMPLE 2 : Mise en évidence de l'absence de cytotoxicité de la composition huileuse conforme à l'invention

Dans cet exemple on a testé la cytotoxicité éventuelle de la composition huileuse conforme à l'invention, telle qu'obtenue par le procédé de l'Exemple 1.

Le test sur la viabilité d'épidermes humains reconstruits utilisé est celui de la réduction au bleu de Formazan (test MTT). L'essai a été réalisé *in vitro* sur épidermes humains reconstruits (« *Reconstructed Human Epidermis* », RHE) modèle RHEps, de surface 0,5 cm$^2$, vendus par la société SkinEthic ®.

Le mélange d'huiles vectrices (huile d'avocat/huile de rosier muscat) dans le rapport massique 3/1 tel qu'utilisé pour l'extraction réalisée à l'exemple 1 a été testé pur (100 %), en application topique sur les épidermes.

La composition huileuse telle que préparée à l'exemple 1 a été testée après dilution à 0,5% (v/v), 1% (v/v), 1,5% (v/v) et 3% (v/v) dans de l'huile de paraffine. Chacune des compositions huileuses ainsi diluées a été appliquée à raison de 5 mg/cm$^2$, sur le stratum corneum des épidermes.

Les mesures de viabilité tissulaires par test MTT ont été effectués après 24 heures d'incubation à 37°C en atmosphère humide air-$CO_2$ (35%/5%), un épiderme non traité servant de témoin.

Les résultats sont reportés dans le tableau I ci-après.

**TABLEAU I**

| Substance | Densité optique à 540 nm | Viabilité (%) |
|---|---|---|
| Aucune (Témoin) | 1,058 ± 0,007 | 100 |
| Huiles vectrices pures | 1,058 ± 0,010 | 100 |
| Composition huileuse à 0,5 % | 1,048 ± 0,013 | 99 |
| Composition huileuse à 1 % | 1,057 ± 0,007 | 100 |
| Composition huileuse à 1,5 % | 1,050 ± 0,006 | 99 |
| Composition huileuse à 3,0 % | 1,031 ± 0,007 | 97 |

Ces résultats montrent qu'aucune diminution significative de la viabilité cellulaire n'est enregistrée après traitement topique avec la composition huileuse conforme à la présente invention dans la gamme de concentrations testées ; ceci comparativement au mélange des deux huiles vectrices testées dans les mêmes conditions qui ne présente lui non plus, aucun effet toxique vis-à-vis des tissus cutanés.

**EXEMPLE 3 : Mise en évidence des propriétés antioxydantes de la composition huileuse conforme à l'invention**

Dans cet exemple on a étudié les propriétés antioxydantes de la composition huileuse conforme à l'invention, telle qu'obtenue par le procédé de l'Exemple 1.

3.1. Principe du test

Le principe du test est basé sur la mesure du degré d'oxydation intracellulaire à l'aide d'une sonde spécifique : le diacétate de 2',7'-dichlorodihydrofluorescéine (DCFH-DA) qui est un indicateur sensible aux espèces réactives de l'oxygène. Le DCFH-DA pénètre dans le milieu intracellulaire où, après clivage des deux groupements acétates par les estérases intracellulaires, le DCFH s'accumule au niveau du cytosol.

L'oxydation intracellulaire du DCFH par différentes espèces réactives de l'oxygène conduit à la formation d'un produit fluorescent le DCF. Plus l'échantillon testé est antioxydant, plus il préviendra l'oxydation du DCFH en DCF et moins le signal de fluorescence sera élevé. À l'inverse, si l'échantillon testé n'est pas antioxydant, la fluorescence se développera autant que dans le contrôle négatif où aucun antioxydant n'est présent. La mesure des intensités de fluorescence permet alors d'évaluer le degré d'oxydation intracellulaire des cellules cutanées soumises à un stress oxydatif (irradiation UV).

3.2. Protocole

L'étude a été réalisée sur des épidermes humains reconstruits *in vitro,* de modèle RHEps, fabriqués par la société SkinEthic. Ils ont été répartis en 7 lots (n=3) :

**Lot 1 : TEMOIN (-) UV :** épidermes non traités ;

**Lot 2 : TEMOIN (+) UV :** épidermes non traités et irradiés ;

**Lot 3 : CONTROLE (-) UV** : épidermes traités avec le mélange d'huiles vectrices (huile d'avocat/huile de rosier muscat : 3/1 en masse) et non irradiés ;

**Lot 4 : CONTROLE (+) UV :** épidermes traités avec le mélange d'huiles vectrices (huile d'avocat/huile de rosier muscat : 3/1 en masse) et irradiés ;

**Lot 5 : TRAITE (+) UV :** épidermes traités avec la composition huileuse préparée ci-dessus à l'exemple 1 (diluée à 0,5%, 1% et 1,5% dans l'huile de paraffine) et irradiés ;

**Lot 6 : TRAITE (+) UV :** épidermes traités avec la composition huileuse préparée ci-dessus à l'exemple 1 (diluée à 0,5%, 1% et 1,5% dans l'huile de paraffine) et irradiés. Ce lot est équivalent au Lot 5 et a été réalisé pour doubler les mesures de façon à avoir au total deux mesures pour chaque concentration testée.

**Lot 7 : CONTROLE POSITIF à la VITAMINE E** : épidermes traités avec de la vitamine E à 3 mM (environ 1,3 mg/ml) dans l'huile de paraffine et irradiés.

A réception, les épidermes ont été transférés dans des plaques de 12 puits contenant du milieu de culture pour épiderme vendu par la société SkinEthic (0,5 ml/puits) et placés à l'incubateur à 37°C, dans une atmosphère à 5% de $CO_2$ saturée d'humidité, pendant 24 heures avant de procéder au traitement des tissus.

Comme indiqué ci-dessus, les propriétés antioxydantes de la composition huileuse conforme à l'invention ont été étudiées après dilution de la composition à 0,5%, 1% et 1,5% dans de l'huile de paraffine.

2,5 $\mu$l de chaque échantillon ont été appliqués en topique sur les épidermes (surface des épidermes : 0,5 cm$^2$). Après traitement, les épidermes ont été replacés à l'étuve à 37°C et incubés pendant 6 heures en atmosphère air/$CO_2$ (95%/5% : v/v). Après incubation, les épidermes ont été rincés avec une solution de HBSS (« *Hanks' balanced salt solution* » : solution saline tamponnée de Hank) puis exposés aux UVB (tubes Philips TL20W/12) dans des plaques 24 puits contenant du tampon phosphate salin (« *phosphate buffered saline* » : PBS).

Après irradiation (200 mJ/cm$^2$), les épidermes ont été transférés dans d'autres plaques contenant du milieu de culture (maintien en survie). L'application des produits a été renouvelée à raison de 2,5 $\mu$l comme précédemment, puis les tissus ont été replacés à l'incubateur, à 37°C, pendant 18 à 24 heures.

A la fin de l'essai, soit 24 heures après l'irradiation aux UVB, les épidermes ont été traités de la façon suivante :

1/ Les membranes poreuses supportant les épidermes ont été détachées de leur support à l'aide d'un scalpel et ont ensuite été placées dans des microtubes où un tampon d'extraction (tampon hypotonique pH 7,5 + saponine) a été ajouté ;

2/ 10 minutes d'incubation à 37°C ;

3/ Centrifugation (force de gravité relative (FGR) : 340g) et prélèvement des lysats ;

4/ Seconde extraction dans les mêmes conditions que ci-dessus ;

5/ Collecte des extraits après centrifugation (FGR : 340g) et mélange des premiers lysats avec les seconds ;

6/ Transfert des échantillons dans des microplaques pour lecture des intensités de fluorescence à l'aide d'un spectrofluoromètre vendu sous la référence Infinité F200 par la société Tecan.

### 3.3. Résultats

Le Degré d'Oxydation Intracellulaire induit par l'exposition aux UVs a été évalué pour chacun des lots. L'intensité de la fluorescence (IF ; moyenne de 3 mesures) mesurée sur le Témoin sert d'étalon et correspond à un DOI de 100 %. Le DOI (%) des lots testés (Contrôle (Lot 4), Traité (Lots 5/6) et Vitamine E (Lot 7)) a ensuite été calculé de la façon suivante :

$$DOI\ (\%) = IF_{\text{Témoin}}\ x\ 100/IF_{\text{Echantillon testé}}$$

où $IF_{\text{Témoin}}$ correspond à l'intensité de la fluorescence du témoin et $IF_{\text{Echantillon testé}}$ correspond à l'intensité de la fluorescence de l'échantillon testé.

Les résultats obtenus sont reportés dans le tableau II suivant :

**TABLEAU II**

| LOTS | Concentration en composition huileuse testée (%) | IF (n=3) | [DOI] (%) |
|---|---|---|---|
| 2 : TEMOIN | - | 2556,0 ± 28,6 | 100 |
| 4 : CONTROLE | huiles vectrices seules | 4348,7 ± 70,8 | 170 [a] |
| 5/6 : TRAITE | 0,5 | 1957,5 ± 19,0 | 77 [a] |
| | 1 | 1835,0 ± 11,4 | 72 [a] |
| | 1,5 | 1660,6 ± 8,5 | 65 [a] |
| 7 : Vitamine E | 0,13 | 335,5 ± 24,5 | 13 |
| [a] : p<0,01 | | | |

**Concernant la composition huileuse conforme à l'invention :**

Les résultats obtenus montrent que le traitement topique des tissus par la composition huileuse conforme à la présente invention permet de réduire le niveau d'oxydation intracellulaire induit par les UV. De plus, il convient de noter que cette activité se manifeste de façon dose-dépendante, dans la gamme des concentrations testées.

Les différences enregistrées au niveau des lots 5/6 correspondant à la composition huileuse diluée à 0,5%, 1% et 1,5% dans l'huile de paraffine sont statistiquement significatives par rapport au Témoin (épiderme non traités) :

- Composition huileuse à 0,5% : 23% de réduction de [D.O.I.] ($p \le 0.01$, test t de Student) ;

- Composition huileuse à 1% : 28% de réduction de [D.O.I.] ($p \le 0.01$, test t de Student) ;

- Composition à 1.5% : 35% de réduction de [D.O.I.] ($p \le 0.01$, test t de Student).

Par ailleurs, en prenant comme témoin les épidermes traités avec le mélange d'huiles vectrices et irradiés (LOT 5), les différences enregistrées sont alors les suivantes :

- Composition huileuse à 0,5% : 55% de réduction de [D.O.I.] ;

- Composition huileuse à 1% : 58% de réduction de [D.O.I.] ;

- Composition huileuse à 1,5% : 62% de réduction de [D.O.I.].

**Comparatif avec la Vitamine E**

La vitamine E a été étudiée à la concentration de 3mM soit 1,3 mg/ml (dilution dans de l'huile de paraffine), soit environ 1444 ppm de vitamine E pure. Cette dose conduit à une activité protectrice vis-à-vis du stress oxydant de 87%.
La composition huileuse conforme à l'invention et telle qu'obtenue suivant l'exemple 1 ci-dessus, contient, avant toute dilution, 411 ppm de vitamine E.

Composition huileuse à 1,5%

1,5% de composition huileuse diluée dans l'huile de paraffine contient 6 ppm de vitamine E et conduit à une protection de 35%.
La vitamine E à 3 mM contient 1444 ppm de vitamine E et conduit à une protection de 87%.
Par conséquent, 240 fois moins de vitamine E conduit à 2,5 fois moins de protection. A concentration égale de vitamine E déposée sur la peau, la composition huileuse conforme à l'invention protège par conséquent 97 fois mieux les cellules cutanées du stress oxydant que la vitamine E pure.

Composition huileuse à 1%

1% de composition huileuse diluée dans l'huile de paraffine contient 4 ppm de vitamine E et conduit à une protection de 28%.
La vitamine E à 3 mM contient 1444ppm de vitamine E et conduit à une protection de 87%.
Par conséquent, 360 fois moins de vitamine E conduit à 3 fois moins de protection. A concentration égale de vitamine E déposée sur la peau, la composition huileuse conforme à l'invention protège 116 fois mieux les cellules cutanées du stress oxydant que la vitamine E pure.

Composition huileuse à 0,5%

0,5% de composition huileuse diluée dans l'huile de paraffine contient 2 ppm de vitamine E et conduit à une protection de 23%.
La vitamine E à 3 mM contient 1444 ppm de vitamine E et conduit à une protection de 87%.
Par conséquent, 722 fois moins de vitamine E conduit à 4 fois moins de protection. A concentration égale de vitamine E déposée sur la peau, la composition huileuse conforme à l'invention protège 191 fois mieux les cellules cutanées du stress oxydant que la vitamine E pure.
L'ensemble de ces résultats démontre d'une part que les polyphénols d'hémérocalle (provenant essentiellement des fleurs) extraits dans le mélange d'huiles vectrices permettent de le stabiliser et d'éviter la dégradation oxydative de ses acides gras sensibles. Ainsi, la qualité des huiles végétales est préservée afin qu'elles puissent apporter tous leurs bienfaits aux cellules cutanées.
Ils démontrent également l'intérêt d'apporter à la peau plusieurs types d'antioxydants à des doses physiologiques plutôt qu'un seul type à forte concentration. En effet, lorsqu'elle est appliquée avant l'irradiation UV, la composition huileuse conforme à l'invention formulée à 0,5%, 1% ou 1,5% protège les cellules cutanées des effets délétères induits par les UV de façon plus efficace que de la vitamine E pure. La composition huileuse conforme à la présente invention comprend une combinaison d'agents antioxydants qui agissent de façon synergique contre les phénomènes oxydatifs auxquels les cellules cutanées sont exposées. Elle apporte à la peau à la fois des agents antioxydants apolaires comme la vitamine E et les caroténoïdes (lutéine, zéaxanthine), mais aussi des agents antioxydants plus polaires tels que les polyphénols (catéchines, quercétine, rutine, acides chlorogéniques) ou la vitamine C. Ceci permet une meilleure protection contre l'oxydation responsable du vieillissement cutané.

**EXEMPLE 4 : Crème contour des yeux anti-âge**

Par les techniques usuelles, on a préparé une crème légère contour des yeux ayant la composition massique indiquée ci-après.

- EDTA tetrasodique 0,1 g
- Caféine 0,2 g
- Glycérine 3,0 g
- Mélange composé d'un copolymère hydroxyéthyle acrylate/sodium acryloyl-diméthyl taurate (SEPINOV EMT10 ; société SEPPIC), d'isostéarate de Sorbitan et de Polysorbate 60 1,0 g
- Huile de rosier muscat 3,0 g
- Oléate de décyle 3,0 g
- Tocophérol 0,7 g
- Composition huileuse de l'exemple 1 1,0 g
- Système de conservation 0,8 g
- Parfum 0,5 g
- Eau qsp 100,0 g

Ce contour des yeux peut être utilisé une à deux fois par jour pendant une durée de 1 à 6 mois.

**EXEMPLE 5 : Crème peau sèche anti-âge**

Par les techniques usuelles, on a préparé une crème anti-âge ayant la composition massique suivante :

- EDTA tetrasodique 0,1 g
- Caféine 0,2 g
- Glycérine 3,0 g
- Polymère réticulé d'acrylate/$C_{10-30}$ alkyle acrylate vendu sous la dénomination commerciale Carbopol® Ultrez 21 par Gattefossé 1,0 g
- Mélange d'alcool arachidylique, d'alcool behénique et de glucoside d'arachidyle vendu sous la dénomination Montanov® 202 par SEPPIC 4,0 g
- Huile de noisette 6,0 g
- Beurre de karité 2,0 g
- Oléate de décyle 3,0 g
- Tocophérol 0,7 g
- Composition huileuse de l'exemple 1 2,0 g
- Système de conservation 0,8 g
- NaOH 0,08 g
- Parfum 0,5 g
- Eau qsp 100,0 g

Cette crème anti-âge peut être utilisée une à deux fois par jour par application sur les zones de la peau à traiter.

**EXEMPLE 6 : Sérum anti-âge**

Par les techniques usuelles, on a préparé un sérum anti-âge ayant la composition massique suivante :

- EDTA tetrasodique 0,1 g
- Extrait de plancton vendu sous la dénomination EPS Seafill par la société CODIF 3,0 g
- Glycérine 3,0 g
- Carbomer vendu sous la dénomination Carbopol® Ultrez 10 par la société Gattefossé 0,2 g
- Glutamate de stéaryle sodique 0,5 g
- Huile de rosier muscat 2,0 g
- Oléate de décyle 1,0 g
- Copolymère réticulé à base de polydiméthysiloxane vendu sous la dénomination commerciale DC9041 par la société Dow Corning 1,0 g
- Tocophérol 0,7 g
- Composition huileuse de l'exemple 1 5,0 g
- Hyaluronate de sodium 0,3 g
- Soude 0,15 g
- Système de conservation 0,8 g

- Parfum 0,5 g
- Eau qsp 100,0 g

Ce sérum anti-âge peut être utilisé une à deux fois par jour par application sur les zones de la peau à traiter.

**Revendications**

1. Composition huileuse à base d'un extrait d'hémérocalle, ladite composition étant **caractérisée en ce qu'**elle comprend un extrait lipophile *d'Hemerocallis fulva*, et un véhicule huileux comprenant au moins une huile végétale.

2. Composition selon la revendication 1, **caractérisée en ce que** ledit extrait est obtenu à partie des parties aériennes *d'Hemerocallis fulva.*

3. Composition selon l'une quelconque des revendications précédentes, **caractérisée en ce que** ledit extrait est obtenu par extraction à l'abri de l'air.

4. Composition selon l'une quelconque des revendications précédentes, **caractérisée en ce que** le véhicule huileux comprend au moins une huile végétale naturelle choisie parmi l'huile d'avocat, l'huile de rosier muscat, l'huile de noisette, l'huile de colza, l'huile de macadamia, l'huile d'argan, l'huile de tournesol, l'huile de cameline, l'huile de bourrache, l'huile d'amande douce, l'huile de carthame, l'huile de calophyllum et leurs mélanges.

5. Composition selon la revendication 4, **caractérisée en ce que** l'huile végétale naturelle est choisie parmi l'huile d'avocat, l'huile de rosier muscat et leurs mélanges.

6. Composition selon la revendication 5, **caractérisée en ce que** le véhicule huileux comprend un mélange d'huile d'avocat et d'huile de rosier muscat.

7. Composition selon la revendication 6, **caractérisée en ce que** le rapport massique huile d'avocat/huile de rosier muscat au sein de la composition huileuse varie de 4/1 à 1/1.

8. Composition selon l'une quelconque des revendications précédentes, **caractérisée en ce que** la teneur en extrait lipophile d'hémérocalle varie de 5 à 20 ppm.

9. Composition cosmétique à application topique, **caractérisée en ce qu'**elle comprend, à titre d'agent anti-oxydant, au moins une composition huileuse telle que définie à l'une quelconque des revendications 1 à 8.

10. Composition cosmétique selon la revendication 9, **caractérisée en ce que** la teneur en composition huileuse varie de 0,1 à 10% en masse par rapport à la masse totale de la composition.

11. Composition cosmétique selon la revendication 10, **caractérisée en ce que** la teneur en composition huileuse varie de 0,5 à 4,0 % en masse, par rapport à la masse totale de la composition.

12. Composition cosmétique selon l'une quelconque des revendications 9 à 11, **caractérisée en ce qu'**elle contient en outre un ou plusieurs actifs secondaires choisis parmi un agent anti-âge complémentaire, l'acétyl tetrapeptide-5, un extrait de plancton, un extrait de maca, un extrait de pétales de coquelicot, une combinaison d'extraits d'anchuse, de coquelicot et de passiflore, un extrait de graines de mimosa, un extrait de pétales de souci, les cellules de cacao, la vitamine C, la vitamine E et l'hexylrésorcinol.

13. Composition cosmétique selon l'une quelconque des revendications 9 à 12, **caractérisée en ce qu'**elle se présente sous forme de gel, d'émulsion, d'émulsion biphasique huile-dans-eau ou eau-dans-huile, d'huile corporelle, de masque, de pommade, d'onguent, de lotion, de solution concentrée, de nanocapsules, de liposomes ou de bâton protecteur pour les lèvres.

14. Composition cosmétique selon l'une quelconque des revendications 9 à 13, **caractérisée en ce qu'**elle comprend en outre des agents de protection contre les rayons ultraviolets ou des pigments formant écran anti-ultraviolet.

15. Composition cosmétique selon l'une quelconque des revendications 9 à 14, destinée à être utilisée pour protéger la peau des agressions de l'environnement.

16. Procédé cosmétique non thérapeutique pour prévenir et combattre les signes du vieillissement cutané, consistant à appliquer sur les zones de la peau concernées au moins une composition cosmétique telle que définie à l'une quelconque des revendications 9 à 14.

17. Utilisation non thérapeutique d'une composition cosmétique à application topique comprenant un extrait lipophile d'hémérocalle et telle que définie à l'une quelconque des revendications 9 à 14, pour prévenir et combattre les signes du vieillissement cutané.

**Patentansprüche**

1. Ölige Zubereitung auf der Basis eines Hemerocallisextrakts, wobei die Zubereitung **dadurch gekennzeichnet ist, dass** sie einen lipophilen Extrakt von *Hemerocallis fulva* umfasst und einen öligen Träger, der mindestens ein Pflanzenöl umfasst.

2. Zubereitung nach Anspruch 1, **dadurch gekennzeichnet, dass** der Extrakt aus den oberirdischen Teilen von *Hemerocallis fulva* hergestellt ist.

3. Zubereitung nach einem der vorangehenden Ansprüche, **dadurch gekennzeichnet, dass** der Extrakt durch Extraktion geschützt vor Luft hergestellt ist.

4. Zubereitung nach einem der vorangehenden Ansprüche, **dadurch gekennzeichnet, dass** der ölige Träger mindestens ein natürliches Pflanzenöl umfasst, das aus dem Avocadoöl, dem Muskatrosenöl, dem Haselnussöl, dem Rapsöl, dem Macadamiaöl, dem Arganöl, dem Sonnenblumenöl, dem Leindotteröl, dem Borretschöl, dem Süßmandelöl, dem Färberdistelöl, dem Calophyllumöl und ihren Gemischen ausgewählt ist.

5. Zubereitung nach Anspruch 4, **dadurch gekennzeichnet, dass** das natürliche Pflanzenöl aus dem Avocadoöl, dem Muskatrosenöl und ihren Gemischen ausgewählt ist.

6. Zubereitung nach Anspruch 5, **dadurch gekennzeichnet, dass** der ölige Träger ein Gemisch aus Avocadoöl und Muskatrosenöl umfasst.

7. Zubereitung nach Anspruch 6, **dadurch gekennzeichnet, dass** das Massenverhältnis Avocadoöl / Muskatrosenöl in der öligen Zubereitung von 4/1 bis 1/1 schwankt.

8. Zubereitung nach einem der vorangehenden Ansprüche, **dadurch gekennzeichnet, dass** der Gehalt an lipophilem Hemerocallisextrakt von 5 bis 20 ppm schwankt.

9. Kosmetische Zubereitung zur topischen Anwendung, **dadurch gekennzeichnet, dass** sie als Antioxidans mindestens eine ölige Zubereitung nach einem der Ansprüche 1 bis 8 umfasst.

10. Kosmetische Zubereitung nach Anspruch 9, **dadurch gekennzeichnet, dass** der Gehalt an öliger Zubereitung von 0,1 bis 10 Ma% in Bezug zur Gesamtmasse der Zubereitung schwankt.

11. Kosmetische Zubereitung nach Anspruch 10, **dadurch gekennzeichnet, dass** der Gehalt an öliger Zubereitung von 0,5 bis 4,0 Ma% in Bezug zur Gesamtmasse der Zubereitung schwankt.

12. Kosmetische Zubereitung nach einem der Ansprüche 9 bis 11, **dadurch gekennzeichnet, dass** sie ferner einen oder mehrere sekundäre Wirkstoffe enthält, die aus einem ergänzenden Anti-Age-Wirkstoff, Acetyl Tetrapeptid-5, einem Planktonextrakt, einem Macaextrakt, einem Extrakt aus den Blütenblättern des Mohns, einer Kombination der Extrakte von Anchusella, Mohn und Passionsblume, einem Extrakt aus den Samen von Mimosen, einem Extrakt aus den Blütenblättern der Ringelblume, den Kakaozellen, dem Vitamin C, dem Vitamin E und dem Hexylresorcinol ausgewählt sind.

13. Kosmetische Zubereitung nach einem der Ansprüche 9 bis 12, **dadurch gekennzeichnet, dass** sie in Form von Gel, Emulsion, Zweiphasen-Öl-in-Wasser- oder -Wasser-in-Öl-Emulsion, Körperöl, Maske, Pomade, Salbe, Lotion, konzentrierter Lösung, Nanokapseln, Liposomen oder Lippenschutzstift vorliegt.

14. Kosmetische Zubereitung nach einem der Ansprüche 9 bis 13, **dadurch gekennzeichnet, dass** sie ferner schützende Wirkstoffe vor ultravioletten Strahlen oder Pigmente umfasst, die einen Schutzschirm gegen ultraviolette Strahlen bilden.

15. Kosmetische Zubereitung nach einem der Ansprüche 9 bis 14, **dadurch gekennzeichnet, dass** sie bestimmt ist, zum Schutz der Haut vor Umwelteinflüssen verwendet zu werden.

16. Nicht therapeutisches kosmetisches Verfahren zur Vorbeugung und zur Bekämpfung von Zeichen der Hautalterung, das darin besteht, auf die betroffenen Bereiche der Haut mindestens eine kosmetische Zubereitung nach einem der Ansprüche 9 bis 14 aufzutragen.

17. Nicht therapeutische Verwendung einer kosmetischen Zubereitung zur topischen Anwendung, umfassend einen lipophilen Hemerocallisextrakt und nach einem der Ansprüche 9 bis 14, um Zeichen der Hautalterung vorzubeugen und zu bekämpfen.

**Claims**

1. Oily composition based on an extract of Hemerocallis, said composition being **characterized in that** it comprises a lipophilic extract of *Hemerocallis fulva* and an oily vehicle comprising at least one vegetable oil.

2. The composition according to claim 1, **characterized in that** said extract is obtained from the above-ground parts of *Hemerocallis fulva.*

3. The composition according to any of the preceding claims, **characterized in that** said extract is obtained by airtight extraction.

4. The composition according to any of the preceding claims, **characterized in that** the oily vehicle comprises at least one natural vegetable oil selected from among avocado oil, rose hip seed oil, hazelnut oil, rapeseed oil, macadamia oil, argan oil, sunflower seed oil, camelina oil, borage oil, sweet almond oil, safflower oil, tamanu oil and mixtures thereof.

5. The composition according to claim 4, **characterized in that** the vegetable oil is selected from among avocado oil, rose hip seed oil and mixtures thereof.

6. The composition according to claim 5, **characterized in that** the oily vehicle comprises a mixture of avocado oil and rose hip seed oil.

7. The composition according to claim 6, **characterized in that** the weight ratio of avocado oil/rose hip seed oil in the oily composition varies from 4:1 to 1:1.

8. The composition according to any of the preceding claims, **characterized in that** the content of lipophilic extract of Hemerocallis varies from 5 to 20 ppm.

9. Cosmetic composition for topical application, **characterized in that** as antioxidant agent it comprises at least one oily composition such as defined in any of claims 1 to 8.

10. The cosmetic composition according to claim 9, **characterized in that** the content of oily composition varies from 0.1 to 10 % by weight relative to the total weight of the composition.

11. The cosmetic composition according to claim 10, **characterized in that** the content of oily composition varies from 0.5 to 4.0 % by weight relative to the total weight of the composition.

12. The cosmetic composition according to any of claims 9 to 11, **characterized in that** it also contains one or more secondary active ingredients selected from among an additional anti-ageing agent, acetyl-tetrapeptide-5, a plankton extract, maca extract, poppy petal extract, a combination of extracts of Anchusa, poppy and Passiflora, an extract of mimosa seeds, marigold petal extract, cocoa cells, vitamin C, vitamin E and hexylresorcinol.

13. The cosmetic composition according to any of claims 9 to 12, **characterized in that** it is in the form of gel, emulsion, biphasic oil-in-water or water-in-oil emulsion, body oil, mask, ointment, unguent, lotion, concentrated solution, nanocapsules, liposomes or lip protection stick.

14. The cosmetic composition according to any of claims 9 to 13, **characterized in that** it further comprises protective agents against ultraviolet radiation or pigments forming an antiultraviolet screen.

15. The cosmetic composition according to any of claims 9 to 14, intended to be used to protect the skin against environmental attack.

16. Non-therapeutic cosmetic method to prevent and combat signs of skin ageing, consisting of applying to the skin regions concerned at least one cosmetic composition such as defined in any of claims 9 to 14.

17. Non-therapeutic use of a cosmetic composition for topical application comprising a lipophilic extract of Hemerocallis and being such as defined in any of claims 9 to 14, to prevent and combat signs of skin ageing.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20110076349 A
- WO 2010112760 A